# EUROPEAN PATENT SPECIFICATION

(11) **EP 1 875 234 B1**
(45) Date of publication and mention of the grant of the patent: **22.06.2011**
(21) Application number: 06722908.8
(22) Date of filing: 25.04.2006
(51) Int. Cl.: G01N 33/50, G01N 33/543

(54) **BIOSURFACE STRUCTURE ARRAY**
OBERFLÄCHENSTRUKTUR-ARRAY
RÉSEAU DE STRUCTURE DE BIOSURFACE

(30) Priority: 26.04.2005 DK 200500610; 01.07.2005 DK 200500981; 27.04.2005 US 675096 P; 19.07.2005 US 700306 P
(43) Date of publication of application: 09.01.2008
(73) Proprietor: Aarhus Universitet, 8000 Århus C (DK)
(72) Inventor: BESENBACHER, Flemming, DK-8210 Århus V (DK); DUCH, Mogens, Ryttergård, DK-8240 Risskov (DK); FOSS, Morten, DK-8660 Skanderborg (DK); PEDERSEN, Finn, Skou, DK-8210 Århus V (DK)
(74) Representative: Schwarze, Holm
(86) International application number: PCT/DK2006/000216
(87) International publication number: WO 2006/114097

(56) References cited:
- S. TURNER ET AL.: "Cell attachment on silicon nanostructures" JOURNAL OF VACUUM SCIENCE AND TECHNOLOGY: PART B., vol. 15, no. 6, 1997, pages 2848-2854, XP002364124 USAVS / AIP, MELVILLE, NEW YORK, NY. cited in the application
- CHESMEL K D ET AL: "CELLULAR RESPONSES TO CHEMICAL AND MORPHOLOGIC ASPECTS OF BIOMATERIAL SURFACES. II. THE BIOSYNTHETIC AND MIGRATORY RESPONSE OF BONE CELL POPULATIONS" JOURNAL OF BIOMEDICAL MATERIALS RESEARCH, WILEY, NEW YORK, NY, US, vol. 29, September 1995 (1995-09), pages 1101-1110, XP008058327 ISSN: 0021-9304
- CHESMEL K D ET AL: "CELLULAR RESPONSES TO CHEMICAL AND MORPHOLOGIC ASPECTS OF BIOMATERIAL SURFACES. I. A NOVEL IN VITRO MODEL SYSTEM" JOURNAL OF BIOMEDICAL MATERIALS RESEARCH, WILEY, NEW YORK, NY, US, vol. 29, September 1995 (1995-09), pages 1089-1099, XP008058334 ISSN: 0021-9304 cited in the application

## Description

### Technical Field

The present invention relates to the identification and analysis of biocompatible materials and structures, in particular materials and structures that affect a cellular function such as direct cellular growth, expansion, differentiation, dedifferentiation, mineralization, intra- or intercellular organization, etc.

### Background

Physiologically relevant cell culture models for high-throughput screening constitute a major bottleneck for the improved treatment of many human diseases. Some physiological and patho-physiological conditions can be reproduced in 3-dimensional cell and organ cultures, but they are not optimal for quantitative readout in a high throughput setting. Many 2-dimensional culture systems for specialised cell types depend upon co-cultivation with a layer of feeder cells, which has the major drawback of inconvenience and irreproducibility. The immobilisation, isolation, expansion, differentiation and culture of living cells on a substrate is thus highly desirable for a variety of biotechnological assays, in particular for cell replacement and the screening and validation of candidate drugs. Microchips to which cells can be surface attached would facilitate high throughput screening of many candidate drugs simultaneously. The ability of a candidate drug to modulate biochemical or physiological pathways implicated in a given disease state could be examined using microchips on which cells are selectively immobilised or attached.

US 2004/0053334A1 patent application describes a substrate comprising a first layer including a heater, and a second temperature-responsive layer that exists in two states, whereby at temperatures below its 'lower temperature solution state (LCTS)' it exhibits a non-cell binding state, while when heated above its LCTS it exhibits a cell-binding state.

Surgical treatment often requires the introduction of implants, for example prostheses, and implantable devises, where the need to overcome a body's own rejection mechanisms and to support tissue regeneration presents a formidable challenge. The clinical success of an implant can thus depend of the cellular behaviour in the immediate vicinity of the interface between an implant and the host tissue. A key element in the progress in this field relies on the identification and use of biocompatible materials for the fabrication of these devises.

Chesmel and Black (1995) J Biomedical Materials Res. 29:1089-1099, describe a system for *in vitro* examination of the response of cells to changes in the chemistry and morphology of polystyrene tissue culture surfaces. The system provides 9 model biomaterial surfaces formed by polystyrene replicas (with different styrene monomer content) caste on micromachined silicon wafers, with radial surface grooves of varying depth (none; 0.5-, or 0.5-µM). Turner et al (1997) J Vac Sci Technol B15: 2848-2854 describe the effect of textured silicon surfaces on the attachment of astrocytes from the central nervous system, employing silicon substrates with two surface textures consisting of a silicon grass and a wet-etched surface that are organised in alternating rows.

In the ongoing search for improved biocompatible materials and structures there remains a need for a high-throughput screening tool to identify structures and surfaces that are optimal for a specific type of cell or tissue.

### Summary

These and other needs are addressed by providing a screening tool comprising a biosurface structure array (BSSA) comprising a number of tester areas whereby each area has a surface topology nano- and/or micrometer scale features.

The present invention is based on the recognition that cellular functions like direct cellular growth, expansion, differentiation, dedifferentiation, mineralization, intra- or intercellular organization, etc, are strongly influenced by the cell's microenvironment. While not wishing to be bound by theory, it is thought that proliferation and differentiation of a specific cell type in vitro and in vivo may depend on the provision of a suitable structure, e.g. a completely artificial structure with no resemblance to structures observable in nature, or structures whose surface properties mimic those of the natural environment of the desired type of cell or tissue, to which the cell can attach. In particular the invention recognises that the 2- and 3-dimensional architecture, or topology, of surfaces in the microenvironment of a cell, is a critical factor in determining cell growth and differentiation. There are a myriad of different microenvironments in which different cell types proliferate and differentiate in vivo, and hence the specific microenvironment that will foster the growth of any specific cell type is difficult and time consuming to determine.

Hence there is a need for a high-throughput screening tool to test different microenvironments for cell cultivation, and in particular to test structures having different surface topology. The manufacture of suitable structures, e.g. artificial structure or structures that mimic surface architectures observed in nature, requires techniques capable of defining features that have micrometer scale or nanometer scale dimensions. The present invention exploits the tools presently developed within nanotechnology, which allow the design and construction of structures whose surface architecture may have a lateral feature size as small as approximately 5nm. In particular, the use of these techniques allows the manufacture of a vast array of different surface topologies each of which is precisely defined, whereby any selected topology can be precisely reproduced in later relevant applications.

The invention provides a biosurface structure array comprising a plurality of tester areas on a single structure, such as a wafer. In order to maximise the number of tester areas per unit area of the wafer, and thereby increase its efficiency as a screening tool, it is desirable to minimise the dimensions of each tester area. Based on the recognition that an individual cell in the body sees its surrounding tissue architecture at the level of micro- and nanostructures, embodiments of the present invention provide a reproduction of such micro- and nanopatterns on a 2-D platform for screening purposes. The identification of a surface structure well suited for obtaining a 2-D layer of a defined type of cells or eventually a structure that best supports the uptake of drugs into the cells will be based upon high-throughput screening of wafers with surface structure arrays (or gradients).

According to one aspect, a key determinant of the minimal required dimensions of a tester area, are the number of cells required in order to attain a statistically significant screening assay, and that may be accommodated per tester area. It has turned out that a statistically significant screening result is achieved when each of the tester areas has an area of at least 0.0625 mm², wherein one linear dimension is at least 250 µm, corresponding to 5 cells of diameter 50 µm each, thereby avoiding that edge effects at the border of tester areas have a statistically significant influence. In particular, the attachment of cells located near/at the perimeter of the tester area is influenced by the difference in affinity of such cells to the respective topological structures of the tester area and the adjacent tester area. Hence, the attachment of the cells to either one of the adjacent areas is influenced by the migration of cells from one tester area to the other. By suitably selecting the lower size limit of each tester area, such edge effects are prevented from having a statistically significant influence on the screening results. The ratio of cross-sectional cell size to surface area of a tester area should preferably be no less than 1/25, allowing a tester area to accommodate an array of 5×x5 cells, i.e. 3x3 cells in the bulk and one row of cells along the perimeter.

According to another aspect, a biosurface structure array for screening cells comprises a plurality of tester areas, wherein each tester area has a surface to be exposed to said cells, wherein the surface has a predetermined periodic topographical structure. When the topographical structure is periodic along one or more lateral directions, e.g. one or more directions parallel with respective edges of the tester area, the structure has a high degree of uniformity across the tester area, thereby increasing the reproducibility of the screening results. Furthermore, periodic structures may be defined/classified by a small number of parameters, thereby allowing a systematic comparison, and a high degree of reproducibility. In some embodiments a cross section of a tester area along one of said lateral directions may be described by a periodic function.

### Description of chemical composition of BSSA

Embodiments of a biosurface structure array include a substrate layer, e.g. in the form of a wafer, and, optionally, a surface layer.

When the substrate layer is silicon, e.g. in the form of a silicon wafer, existing manufacturing techniques for generating a patterned surface structure may be employed. Other suitable base materials for the preparation of the biosurface structure array include any semiconductor (doped or not-doped), a single metal, an alloy, a ceramic, a polymer, a co-polymers, a composite, a drug delivery system, e.g. a polymer with bioactive molecules, other bioactive compounds or any combination thereof.

In embodiments of the invention, the surface layer comprises a material that supports the appropriate response from the cells to be cultured. More specifically, the material needs a suitable degree of biocompatibility. Examples of surface layers include a metallic surface deposit, e.g. tantalum, titanium, Ti-AI-V alloys, gold, chromium, metal oxides, semiconductor oxides, metal nitrides, semiconductor nitrides, polymers, biopolymers, or other alloys.

In some embodiments, the biosurface structure array comprises additional components such as one or more bioactive compound, e.g. a polymer comprising a polypeptide, deposited on a surface layer of said array, adsorbed bioactive polymers or compounds, and/or the like. For example, said polymer may be selected from the group consisting of an antibody, antigen, glycoprotein, lipoprotein, DNA, RNA, polysaccharide, lipid, organic compound, inorganic compound

### Structural properties of BSSA

A surface of the wafer is divided into a plurality of tester areas, i.e. sub-areas of the total surface area of the wafer. The surface of each tester area includes a regular nano- or micrometer scale structure along one dimension or two dimensions within the surface plane of a tester area. Examples of structures with nano- or micrometer scale features in one dimension include features that extend over the entire length of the tester area in a longitudinal direction and have nano- or micrometer scale dimensions in a lateral direction. Examples of two dimensional structures include structures including features having nano- or micrometer scale dimensions in two directions within the plane defined by the surface of the tester area.

The topographical structure is regular, i.e. the structure comprises features that are arranged in a predetermined ordered, non-stochastic pattern, thereby ensuring the reproducibility of the screening results. It is a further advantage that regular topographical structures of micro and/or nanometer scale can be reproducibly produced by known production techniques, such as photolithography, e-beam lithography, hot embossing, nano-imprint lithography, microcontact printing, focussed ion beam etching, etc. Other examples of production steps that may be involved in the production of the biosurface array include chemical etching, laser ablation, plasma spray coating, abrasive blasting, engraving, scratching, micro-machining, etc.

When the topographical structure is periodic along one or more lateral directions, e.g. one or more directions parallel with respective edges of the tester area, the structure has a high degree of uniformity across the tester area, thereby increasing the reproducibility of the screening results. Furthermore, periodic structures may be defined/classified by a small number of parameters, thereby allowing a systematic comparison. In some embodiments a cross section of a tester area along one of said lateral directions may be described by a periodic function.

The term nanometer scale as used herein is intended to refer to a length scale in the range of between about 1 nm and about 1000nm, in particular between about 1 nm and about 100nm. The term micrometer scale as used herein is intended to refer to a length scale in the range of between about 1µm and about 1000µm.

In embodiments of the invention the lateral dimensions of the topographical features in at least one lateral direction are within the interval 1-10⁵ nm, e.g. 1-2 nm, 2-4 nm, 4-10 nm, 10-100 nm, 100-1000 nm, 1-2 µm, 2-4 µm, 4-10 µm, 10-100 µm.

In some embodiments, the scale of the features varies from tester area to tester area, e.g. some tester areas have features on a nanometer scale, i.e. between 1-1000nm, while other tester areas have features at micrometer scale, i.e. between 1-1000µm. Similarly, each tester area may comprise features at different scales, e.g. a structure with a small period along one direction and a larger period, e.g. by a factor of 10, 100, or 1000 larger, in another direction. In some embodiments a structure may even have features at different scales along the same direction, e.g. features at a small scale superimposed features at a larger scale. For example, a wafer with tester areas having regular nanometer or micrometer scale structures as described herein may subsequently be processed so as to superimpose an irregular/random structure on the surface of some or all of the tester areas,
e.g. a so-called silicon grass structure as described by Turner et al. (1997) (ibid.)

The depth/height of the features, i.e. their linear dimension in a direction projecting out of the surface of the tester area may likewise be on the nano-or micrometer scale, i.e. the structures may have heights/depths in the range 1nm - 1000µm. In some embodiments the structures have a depth/height of greater than 0.6 µm, preferably between 1.2 µm and 1000.0 µm, and more preferably at least 1.2 µm, 2.0 µm, 5.0 µm, 10 µm, 20 µm, 50 µm, 100 µm or 1000µm.

In some embodiments, the structures may further include patterns along the direction projecting out of the surface. For example, the protruding surfaces may be structured by a chemical or other process, e.g. a chemical etching process.

In some embodiments, the period of the periodic structure is smaller than the linear dimension of the tester area, e.g. the linear dimension of the tester area is an integer multiple of the period of the structure.

In embodiments of the invention, the surface structure comprises individual features, such as protrusions, pillars, ridges, channels, recesses, holes, etc. of predetermined lateral dimension and height/depth. Alternatively, a feature of the topological structure may be defined as a period of the periodic structure. Hence, the lateral dimensions of a feature of a periodic structure may be defined as the period of the periodic shape/function, i.e. the length of the shortest interval over which the structure repeats its shape.

The lateral cross section of each of the features may have any shape with a suitably defined circumference and/or geometrical shape, such as square, rectangular, hexagonal, polygonal, round, circular, etc., or a combination thereof. The top and/or side surfaces of the features may be substantially flat, curved and/or structured, e.g. modified by a chemical or other process. Different features may have the same or different shapes.

In embodiments of the invention, the smallest lateral/vertical dimension of the structure is smaller or at least comparable to the linear dimension of the organisms/cells to be exposed to the surface, such that the organisms experience a structure at a scale smaller or at least comparable to their own size.

The wafer on which the tester areas are located may have any suitable size, e.g. a 2, 4, 6, 8, 12, 14, or 16 inch wafer, or an even smaller or larger wafer, thereby providing biosurface structure arrays of different sizes and allowing parallel screening of both small and large diversities of different structures.

The tester areas may have any geometrical shape, such as circular or polygonal, e.g. hexagonal. Nevertheless, rectangular tester areas, in particular square tester areas, are convenient and provide an efficient utilisation of the wafer area. Square tester areas further provide a small boundary relative to the area of the tester area and, at the same time, an efficient utilisation of the wafer area. Hence, a large effective area for screening is provided while limiting the boundary effects at the edges of the tester areas.

The tester areas may have the same or different dimensions along each lateral direction. In embodiments of the invention, the dimension of the tester areas in each direction is at least 0.25mm, preferably at least 0.3mm, more preferably at least 0.5mm, most preferably at least 1 mm, e.g. in the interval between 0.25mm and 25mm, preferably between 0.3mm and 20mm, more preferably between 0.5mm and 10mm, most preferably between 1 mm and 5mm, e.g. the tester areas may be squares with dimension of about 2mm × 2mm, 5mm x 5mm, 10x10mm, 0.25x0.25mm, or the like.

In one embodiment each tester area has dimensions of between about 2mmx2mm to about 10mmx10mm providing between about 60 to about 1500 tester areas on a standard 4 inch wafer. It is an advantage that a screening of cells with a size of approximately 50µm x 50µm can be conducted with a high statistical significance by providing tester areas of 2mm x 2mm, where potentially circa 1600 cells can be accommodated per tester area.

When the surface area is at least 0.09 mm², and wherein each linear dimension is at least 300 µm, at least about 16 cells of dimension 50µm x 50µm can be accommodated in the bulk of the tester area, surrounded by at least one row of cells along each edge of the tester area. Hence, the tester area is sufficiently large to provide a uniform microenvironment for 16 cells without border effects. When the surface area is at least 0.1225 mm², and wherein each linear dimension is at least 350 µm, at least about 9 cells of dimension 50µm x 50µm can be accommodated in the bulk of the tester area, surrounded by at least two rows of cells along each edge of the tester area, thereby further reducing the influence of edge effects. Alternatively, when the surface area is at least 0.1225 mm², and wherein each linear dimension is at least 350 µm, at least about 25 cells of dimension 50µm x 50µm can be accommodated in the bulk of the tester area, surrounded by at least one row of cells along each edge of the tester area, thereby increasing the number of cells available for analysis.

Embodiments of the present invention can be implemented in different ways, including the biosurface structure array described above and in the following, a method of screening for a biocompatible surface structure, further product means, and one or more uses thereof, each yielding one or more of the benefits and advantages described in connection with the first-mentioned aspect, and each having one or more embodiments corresponding to the embodiments described in connection with the first-mentioned aspect and/or disclosed in the dependent claims.

In particular, the invention further relates to a kit comprising a biosurface structure array described herein. Such a kit may be used for one or more of the following: A cell attachment assay, a cell differentiation assay, a cell spreading assay, a cell proliferation assay.

### A kit comprising a BSSA suitable for cell screening:

A kit comprising a BSSA suitable for screening one or more cellular effects of the surface structures on the wafer may comprise: One or more BSSA wafers, and optionally may include standard cell media, sterile culture containers, or other standard culture items, standard cells, cells labelled with fluorescent dyes, and/or the like. Preferably, the size of the tester areas on the one or more BSSAs is selected such that each tester area can accommodate at least 25 of the cells dimension 50µm or of the cells included in the kit, preferably at least 50 cells, more preferably at least 100 cells, most preferably at least 1000 cells.

For example, such a kit is suitable for measuring the ratio between attachment of two cell types. Accordingly in this example, the kit may include or be used to screen cells labelled with e.g. the fluorescent proteins EYFP and ECFP. The fluorescent proteins can be expressed in the cells using standard gene transfer techniques. To facilitate the monitoring of the cell numbers of the two different cell types the fluorescent proteins can further be localized to the nucleus.

### A device for analysing a BSSA:

The results of screening with a BSSA wafer may be analysed by a variety of methods including an optical inspection by means of a microscope. When the microscope is placed in an incubator capable of controlling humidity, CO₂, temperature and/or other parameters, a controlled environment is provided for the cell cultures on the BSSA. An embodiment of a device suitable for automatic or at least semi-automatic acquisition of screening results comprises a microscope positioned in an incubator, a support for receiving a BSSA wafer, an image recording device, and a control unit for controlling the position of the wafer relative to the optical axis. The control unit is further adapted to control the microscope to acquire one or more images of each tester area. The control unit further comprises an image processing unit adapted to receive and the recorded images and to perform an image analysis of the received images. For example, the image analysis may include the detection of cells of different fluorescent colours and the calculation of the ratio of the number of cells of different fluorescent colours. Alternatively or additionally, the image analysis process may include a measurement of the total area of a tester area covered by cells with a given fluorescent colour, or similar image analysis steps.

### Use of kit comprising a BSSA in a screening assay

The invention further relates to a method of screening for a biocompatible surface structure, comprising
(a) combining a biosurface structure array as described herein with isolated cells or organisms under conditions suitable for the maintenance and/or growth of said cells or organisms.

Using such BSSA technology enables screening of a large number of structures in one single experiment by either a single or a mixed cell culture. The biosurface structure array described herein may be used for assaying a large variety of screening parameters localized to the individual tester areas on the BSSA wafer including: mineralization, differentiation markers on the cells, cell number (enhanced or decreased), gene induction in reporter cells, bio fouling, attachment and expansion of a specific cell type, etc.

Accordingly, in some embodiments, the method further comprises comparing the frequency of attachment of said cells or organisms to any one tester area of said biosurface structure array, thereby determining differences of attachment of cells to the respective surface structure of the different test cells. In another embodiment, the method further comprises comparing spreading of said cells or organisms on any one tester area of said biosurface structure array. In yet another embodiment, the method further comprises comparing differentiation of cells or organisms attached to any one tester area of said biosurface structure array.

In some embodiments, the method further comprises incubating (a) under conditions suitable for the maintenance and/or growth of said cells or organisms.

The biosurface structure array described herein may be used for detecting/testing different microenvironments for cultivation of a large variety of cells or organisms, including cells, such as animal cells, plant cells, mammalian cells, yeast cells, eukaryotic or prokaryotic cells or organisms, etc.

When each linear dimension of the tester area is large enough to accommodate a row of at least 5 cells, preferably at least 10 cells, more preferably at least 20 cells, the tester area is large enough to accommodate a sufficiently large number of cells in the bulk area, i.e. removed from the edge of the tester area, to provide a screening parameter result of sufficient statistical relevance.

The invention further relates to a biocompatible material having a surface, wherein at least a part of said surface is characterized by a topology comprising nanometer scale or micrometer scale features, the surface being obtained/obtainable by the method described above and in the following.

The invention further relates to a wafer comprising a biosurface structure array described herein, and use of such a wafer in an *in vitro* cell bioassay. The invention further relates to a kit comprising such a wafer for performing an *in vitro* cell bioassay.

### Brief description of the drawings:

The invention will be explained more fully below in connection with embodiments and with reference to the drawings, in which:
Fig. 1 shows a top view of an example of a biosurface structure array wafer.
Fig. 2 shows a cross sectional view of the wafer of fig. 1 along the line A-B.
Fig. 3 shows a top view of another example of a biosurface structure array wafer.
Fig. 4 shows a top view (Fig. 4a) and a cross-sectional view (Fig. 4b) of a topological structure comprising alternating trenches of width X (in µm) and ridges of width Y (in µm).
Fig. 5 shows a top view (Fig. 5a) and a cross-sectional view (Fig. 5b) of a topological structure comprising square holes and a predetermined pitch distance.
Fig. 6 shows a top view (Fig. 6a) and cross-sectional views (Fig. 6b,c) of a topological structure comprising rectangular holes of dimension X (in µm)×Y (in µm) separated with ridges of width X µm.
Fig. 7 shows a top view (Fig. 7a) and a cross-sectional view (Fig. 7b) of a topological structure comprising pillars of a predetermined dimension and a predetermined pitch distance.
Fig. 8 shows a top view of a topological structure comprising alternating square holes and pillars.
Fig. 9 shows a tester area accommodating 25 cells.
Fig. 10 shows a tester area accommodating 49 cells.
Fig. 11 shows top views of examples of topological structures with circular pillars.
Fig. 12 shows an example of cells labelled with whole cell labelling.
Fig. 13 shows an example of cells labelled with nuclear localized fluorescent proteins.
Fig. 14 shows a schematic block diagram of a device for recording results of a screening for a biocompatible surface structure.
Fig. 15 illustrates an example of a gene induction assay.
Fig. 16 shows embryonic fibroblasts cultured on a reference surface of a BSSA wafer.
Fig. 17 shows embryonic fibroblasts cultured on a tester area having a "D2/4" surface structure.
Fig. 18 shows embryonic fibroblasts cultured on a tester area having a "D2/4" surface structure.
Fig. 19 shows embryonic fibroblasts cultured on a tester area having a "D2/10" surface structure.
Fig. 20 shows MC3T3 cells cultured on glass and on a reference surface of a BSSA wafer.
Fig. 21 shows MC3T3 cells cultured on a tester area having a "D2/4" surface structure.
Fig. 22 shows MC3T3 cells cultured on a tester area having a "D2/10" surface structure.

### Detailed description

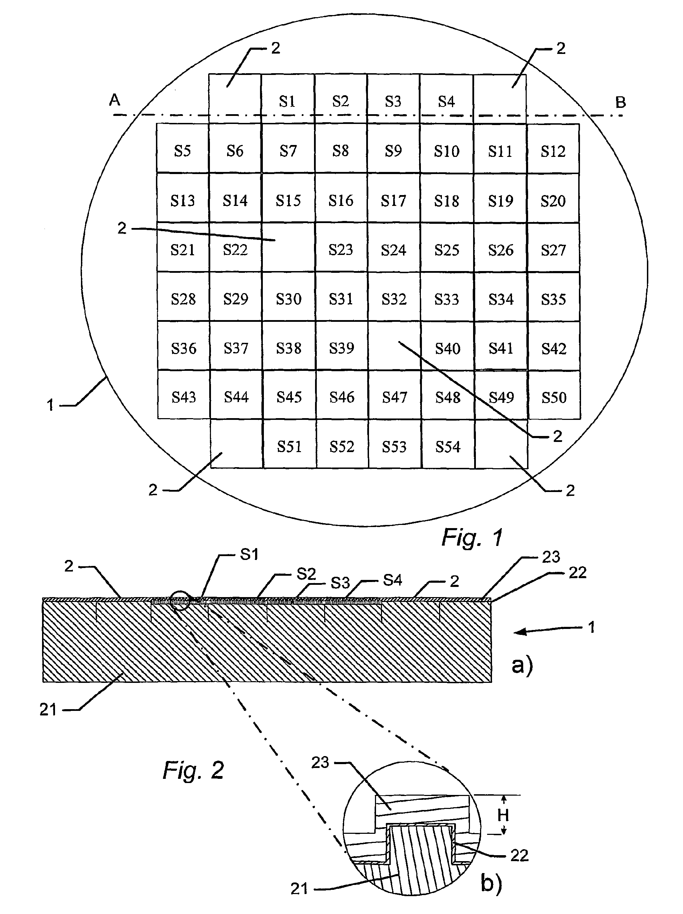

Fig. 1 shows a top view of an example of a biosurface structure array wafer.

The BSSA wafer 100 comprises 60 tester areas. A number of tester areas 2 are left "blank", i.e. they have not been processed to have a structured surface. Consequently, the surfaces of the tester areas 2 are substantially flat. Consequently a control experiment is inherently included in each parallel screening test with the BSSA screening tool. The remaining tester areas, designated S1,S2...,S54, comprise respective structured surfaces as described herein. The tester areas are squares of dimension 10mm x 10mm.

Fig. 2 shows a cross sectional view of the wafer of Fig. 1. Fig. 2a shows a cross section of the entire width of the wafer along the line labelled A-B. Fig. 2b shows an enlarged view of a portion of the surface of one of the tester areas. The wafer 1 has a layered structure including a patterned substrate layer 21, e.g. a silicon layer, and a surface layer 23. The surface of the wafer is patterned, e.g. by a photolithography process, to provide different patterns on the surfaces of the respective tester areas S1, S2, ..., S54. The structures have a depth/height H. In a photolithography process the height H is controllable by the etching process. The patterned surface is covered by a thin layer of silicon dioxide 22, and/or a surface layer 23 of a different biocompatible material such as tantalum or any other metal, metal oxide, metal nitrides, metal carbides, diamond, diamond like carbon, semiconductor, semiconductor oxide/nitride, insulator, polymers, copolymers. Between the tester areas there is a "blank" border area with no structure, i.e. the border area has not been processed to have a structured surface. In this case the thickness of the blank border area is 0.3 mm. However, the thickness could be larger or smaller than 0.3 mm. In some embodiments, there is no border area, i.e. the structures may touch each other directly. A blank border line aids visual alignment and identification of the structures.

It is noted that the fig. 2 is schematic and not drawn to scale. In particular, the vertical dimensions may be exaggerated to improve readability.

### Method of manufacture of a BSSA

A biosurface structure array described herein can be manufactured by a number of production techniques. Examples of procedures for the manufacture of a BSSA include one or more of the following techniques which are known as such in the art:
- Photolithography methods: Photolithography is a process known as such in which geometric shapes/patterns are transferred from a photomask to the surface to be structures, e.g. the surface of a wafer. Photolithography equipment with minimum lateral feature sizes ranging from around 1 micrometer to below 100nm is known as such. Photolithography processes are described in e.g. S.M.Sze: Semiconductor Devices, Physics and Technology, 2nd Edition, John Wiley & Sons 2002, Chapter 12: Lithography and Etching; and in Plummer, Deal, Griffin: Silicon VLSI Technology, Fundamentals, Practice, and Modeling, Prentice Hall 2000, Chapter 5: Lithography.
- E-beam lithography: In principle, E-beam lithography can be used to expose a photoresist in exactly the same way as the light is used in photolithography. E-beam lithography has a particularly high resolution up to around 5nm.
- Hot embossing: Hot embossing uses a master stamp to imprint micro-and nanometer scale structures on polymer substrates. The method allows the master stamp to produce many fully patterned substrates using a wide range of polymer materials. Hot embossing provides a low-cost, highly versatile manufacturing method that is well suited for the manufacture of BSSA for uses ranging from research and development applications to high-volume production. High aspect ratios with a very high degree of homogeneity may be achieved for micro- and nanometer scale structures on large-sized wafers, such as 8 inch or 12 inch wafers. Features sizes below 20nm are possible. The master stamp may be produced by e.g. E-beam lithography techniques.
- Other examples of production steps or processes that may be involved in the production of the biosurface array include nano imprint lithography, laser ablation, chemical etching, plasma spray coating, abrasive blasting, engraving, scratching, micro machining, or the like.

Furthermore, the manufacture of a BSSA may include further modification processes for modifying the surface structures of the tester areas, thereby allowing an even larger range of microenvironments to be screened. Examples of such techniques include but are not limited to topographical modifications directly applicable on 3D, such as various blasting techniques, e.g. sandblasting, microblasting and glassblasting. Further examples of such modification processes include chemical modifications, such as processes resulting in one or more gradients in one or more directions across a wafer, e.g. by protein adsorption, peptide adsorption, chemical deposition, chemical etching, and/or electrochemical deposition. For example, a chemical gradient may be produced by gradually submerging a wafer into a suitable solution. Consequently, on a single wafer, a number of different structures may be screened and, if tester areas with the same structure are repeated along the chemical gradient, each structure is screened under varying chemical conditions. Consequently, a highly parallel screening of different microenvironments is facilitated.

### Example 1.

### Manufacture and testing of a 4 inch BSSA wafer comprising 60 tester areas - screening for a bone cell compatible microstructure.

### A. Manufacture of a BSSA wafer

A single-sided polished silicon wafer (4 inch) with a thickness of 525±25 µm provided a substratum for the manufacture of a biocompatible material. The wafer was an n-type wafer with a resistivity of 1-20 ohm cm. A micrometer-sized pattern was printed onto the polished side of the silicon wafer by standard photolithography and reactive ion etching in a SF₆/O₂ discharge according to the following protocol:
1. The wafers were pre-etched with buffered hydrofluoric acid (BHF, BHF is a solution of concentrated HF (49%), water, and a buffering salt, NH₄F, in about the ratio 1:6:4) for 30 seconds and then dried under N₂ flow, and
2. the wafer was then spin-coated with a 1.5 µm thick layer of photoresist AZ5214 , Hoechst Celanese Corporation, NJ, US (the chemical composition can be found at the Material Safety Data Sheet (MSDS) supplied by Hoechst Celanese Corporation).and pre-baked at around 90°C for 120 seconds, and
3. the photoresist-coated wafer was exposed to UV light for 5 seconds in an EVC aligner, model AL6-2 , through a suitable mask, allowed to develop for 50-60 seconds and then post-baked for 1 minute at 120°C, and
4. the photoresist-coated wafer was then patterned by briefly etching with BHF for approximately 30 sec., and then subjected to Reactive Ion Etching (RIE) at a rate of approximately 0.30µm/minute, and the resist was stripped with acetone followed by RCA cleaning. The RCA cleaning procedure has three major steps used sequentially: Removal of insoluble organic contaminants with a 5:1:1 H₂O:H₂O₂:NH₄OH solution (SC1). Removal of a thin silicon dioxide layer where metallic contaminants may have accumulated as a result of (I), using a diluted 50:1 H₂O:HF solution. Removal of ionic and heavy metal atomic contaminants using a solution of 6:1:1 H₂O:H₂O₂: HCl (SC2).
5. The patterned wafer was then passivated by dry oxidation with a 20 nm SiO₂ layer, thermally grown at 1000°C for 15 minutes.
6. A 250 nm tantalum layer was deposited onto the surface of the patterned wafer by sputter deposition.

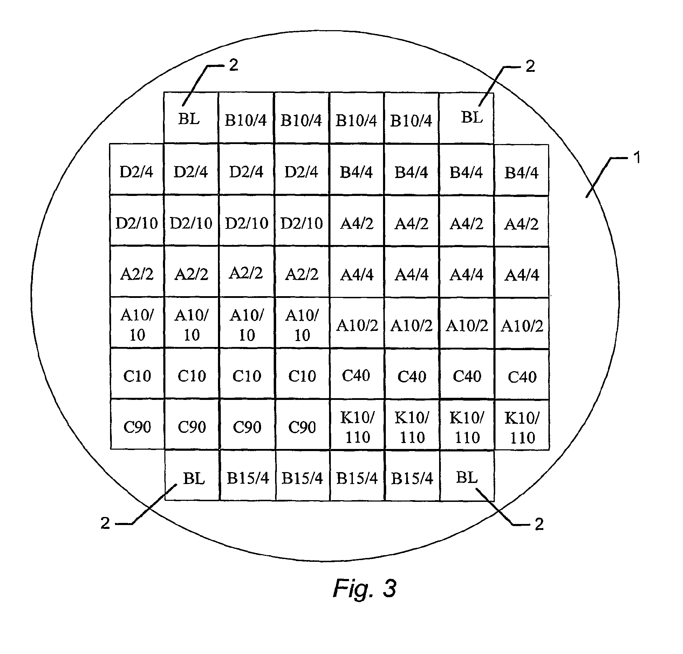

Fig. 3 shows a top view of one of the prepared wafers. Wafers 1 were prepared comprising 60 tester areas, each with a dimension of 10mmx10mm, wherein each area has a specific lateral topology prepared according to Example 1. Each wafer includes four control tester areas 2 having a planar surface, and 4 replicates of each of 14 different lateral topologies. A series of wafers were produced according to these defined parameters, wherein the depth of the lateral topology was defined as either 0.07 µm, 0.25 µm, 0.60 µm, 1.20 µm, 1.60 µm.

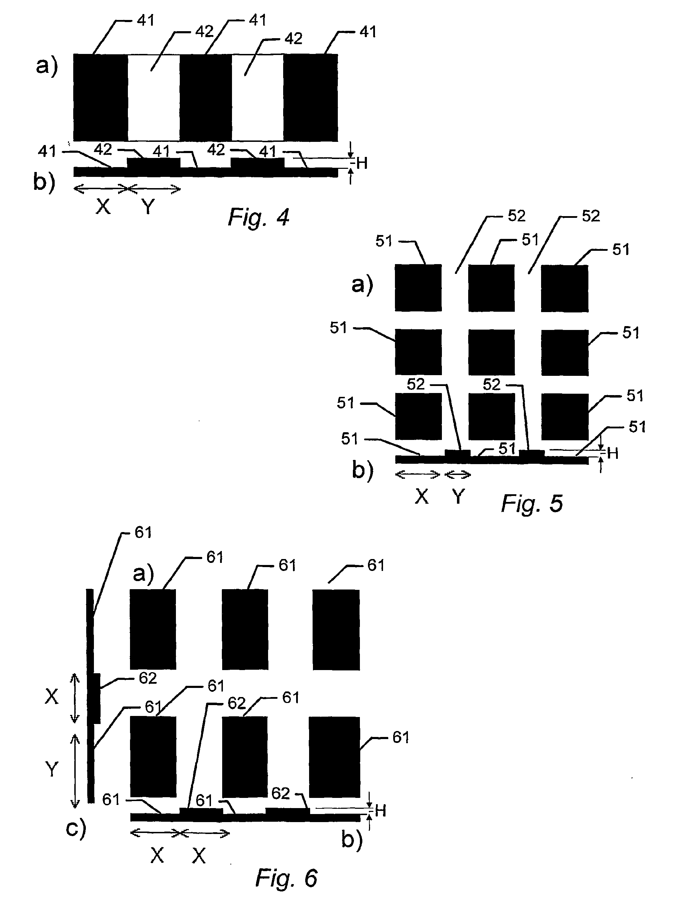

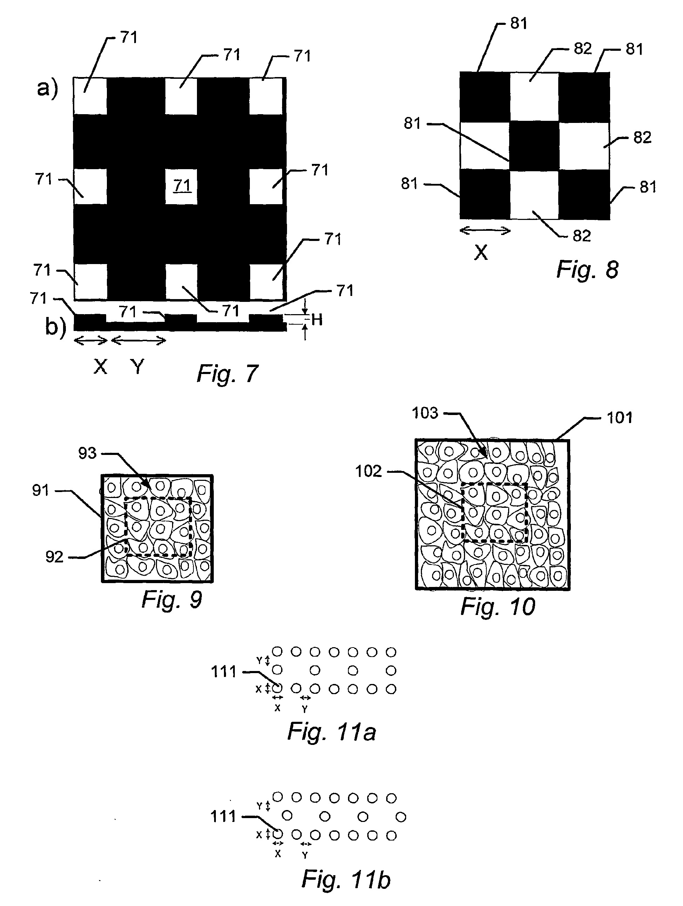

Each specific lateral structure is repeated 4 times. In fig. 3, each tester area is labelled to indicate its topological surface structure, where the labels indicate the following structures:
- "BL": No structure, i.e. a substantially flat surface.
- "AX/Y": Line structures as shown in fig. 4. The structure includes trenches 41 of width X (in µm) and ridges 42 of width Y (in µm). Hence, the line structure of fig. 4 has micrometer scale features along one dimension only. In Example 1, the areas A2/2 include a line structures with trenches of width 2 µm and ridges of width 2µm, the areas A4/4 include line structures with trenches of width 4 µm and ridges of width 4µm, the areas A10/10 include line structures with trenches of width 10 µm and ridges of width 10µm, the areas A4/2 include line structures with trenches of width 4 µm and ridges of width 2µm, and the areas A10/2 include line structures with trenches of width 10 µm and ridges of width 2µm.
- "BX/Y": A square-hole structure as shown in Fig. 5, The structure includes square holes/recesses 51 with dimension X(in µm)×X(in µm) and a pitch distance of X+Y, i.e. the net of ridges 52 have a width of Y. Hence, the structure of fig. 5 has micrometer scale features in both dimensions within the plane of the surface of the tester area. In example 1, the areas B4/4 include square holes with dimension 4µm×4µm and pitch distance 8 µm, the areas B10/4 include square holes with dimension 10µm×10µm and pitch distance 14 µm, and the areas B15/4 include square holes with dimension 15µm×15µm and pitch distance 19 µm.
- "KX/Y": A structure comprising rectangular holes/recesses separated by ridges as shown in Fig. 6. The structure includes rectangular holes 61 of dimension X (in µm)×Y (in µm) separated with ridges 62 of width X (in µm). Hence, the areas K10/110 include rectangular holes with dimension 10µm×110µm separated with ridges of width 10µm. • "DX/Y". A structure comprising protrusions/pillars as shown in Fig. 7. The structure comprises protrusions/pillars 71 with a square cross section of dimension X(in µm)×X(in µm) and a pitch distance of X+Y. Hence the areas D2/4 include a square-pillar structure with pillar dimensions 2 µm×2 µm and a pitch distance of 6 µm, and the areas D2/10 include a square-pillar structure with pillar dimensions 2 µm×2 µm and a pitch distance of 12 µm.
- "CX". A square-hole/pillar structure as shown in fig. 8. The structure has the appearance of a chess board with holes 81 and protrusions/pillars 82, both having the shape of squares with dimension X(in µm)×X(in µm). Hence, the areas C10 include a square-hole/pillar structure with dimension 10µm×10 µm of both holes and pillars, the areas C40 include a square-hole/pillar structure with dimension 40µm×40 µm of both holes and pillars, and the areas C90 include a square-hole/pillar structure with dimension 90µm×90 µm of both holes and pillars.

It is understood that the preparation method described above may also be applied to wafers with other forms and sizes of tester areas as well as other types of structures. The same production process may be used for a variety of different wafers, where the layout of the tester areas and the particular surface structures are determined by the mask through which the wafer is exposed.

### B. Screening a BSSA wafer identifies a biocompatible material for attachment of bone cells (osteoblasts)

Each wafer was placed in a P15 dish (NUNC, Biotech line) and washed with 70% ethanol and then PBS (6.8 g NaCl, 0.43 g KH₂PO₄, 0.978 g Na2HPO4*2H2O in 1 liter double distilled water ~pH 7.4). The wafer was seeded with cells of a MC3T3-E1 murine osteoblastic cell line (Sudo, H et al. 1983, J Cell Biol 96(1):191-98), at a concentration of 20,000 cells/cm². The cells were cultured for 4 days in plain medium (alpha-minimal essential medium [a-MEM], 10% fetal calf serum [FCS], 100U/ml penicillin, and 100 microgram/ml streptomycin (supplied by Gibco, Invitrogen). The cells were maintained in a humidified incubator (5% CO₂/95% air atmosphere at 37°C), and subsequently 50µg/ml ascorbic acid (Wako Chemicals, DE) and 10mM β-glycerophosphate (Sigma-Aldrich, DK) were included in the growth medium. The cells were cultured for 3 weeks, with a change of growth medium twice a week.

### Growth of murine osteoblastic cells on tester areas of wafer:

a) *In vitro* mineralisation. After 3 weeks culture, the wafers from each cell culture dish were tested for mineralization by washing the wafers with PBS and fixing the cells on the wafer with 70% ethanol for 1 hr at - 20°C. The cells were then rinsed in double-distilled H₂O and then stained with 40 mM Alizarin Red S adjusted to pH 4.2 (Sigma-Aldrich, DK) for 10 minutes at room temperature (about 20-25°C). The wafers were post-rinsed with H₂O and incubated in PBS for 15 minutes to reduce non-specific staining.
b) Alizarin Red, that binds to calcium, stained all tester areas weakly, while the test areas having the microstructure D2/4 were strongly stained, as confirmed by cell growth on wafers performed in several independent experiments. D2/4 has a two-dimensional periodic structure of square pillars of dimension 2µm x 2 µm and pitch distance of 6 µm (as decribed in connection with fig. 7).
c) A comparison of mineralisation on wafers with different depths of the lateral topology, revealed a significant vertical dimension dependence for the mineralization process during cell culture. Enhanced mineralization on the test area D2/4, by comparison to the other test areas, was only detected when the vertical dimension of the lateral topology was greater than 0.6µm. Tester areas with a vertical dimension of 1.2 µm and 1.6 µm showed mineralization.

Examples of methods of demonstrating the biocompatible properties of materials include cell attachment assays as described above, a cell spreading assay, a cell motility assay, a differential cell attachment assay, a cell proliferation assay, a gene induction assay, etc.

For example, in a cell proliferation assay cells may be labelled with fluorescent proteins and cell proliferation determined by scanning the BSSA wafer several times for a period lasting up to several days. When the wafer is placed in an automated analysis device including a microscope in an incubator as described herein, the device can perform the scanning in an automated fashion.

Fig. 9 shows a tester area accommodating 25 cells. The square tester area 91 is sufficiently large to accommodate 5×5=25 cells. During analysis of a screening experiment, such as a cell attachment experiment, a cell differentiation experiment, or the like, the cells in the bulk of the tester area are analysed, e.g. counted while the cells along the boundary of the tester area 91 are disregarded in the analysis; they merely serve as an insulation of the cells in the bulk area against the microenvironment of the adjacent tester areas (not explicitly shown). In Fig. 9, the bulk area is illustrated by a dashed square 92 and is large enough to accommodate 3x3=9 cells. Hence, the cells within the square 92 are used for analysis while the cells in the border area 93 surrounding the analysis square 92 provide a screen against border effects.

Fig. 10 shows a tester area accommodating 49 cells. The square tester area 101 is sufficiently large to accommodate 7x7=49 cells. As described above, during analysis, the cells in the bulk of the tester area, illustrated by dashed square 102, are analysed, while the cells in the border area 103 surrounding the analysis square 102 provide a screen against border effects. In the example of fig. 10, nine cells are used for analysis and two rows of screening cells are disregarded along each edge. Alternatively, the centre 25 cells may be used for analysis with only one row of screening/insulating cells along each edge.

Hence, statistically relevant results may be achieved while reducing sources of error caused by gradient effects due to cells close to an edge of a tester area being influenced both by the structure of the tester area and the surrounding structure, e.g. the structure of an adjacent tester area. Larger tester areas can accommodate even more cells, thereby providing more cells for analysis, e.g. 5x5, 10x10, 20x20 cells, 40x40 cells, or even more cells, thereby providing improved statistics. Furthermore, large tester areas allow disregarding of 2, 3, 4 or more rows of cells along the edges, thereby reducing the influence of border effects.

Fig. 11 shows top views of two examples of topological structures with protrusions/pillars having a circular cross-section. The circular pillars have a diameter x and a pitch distance y, as illustrated in figs. 11a and 11b.

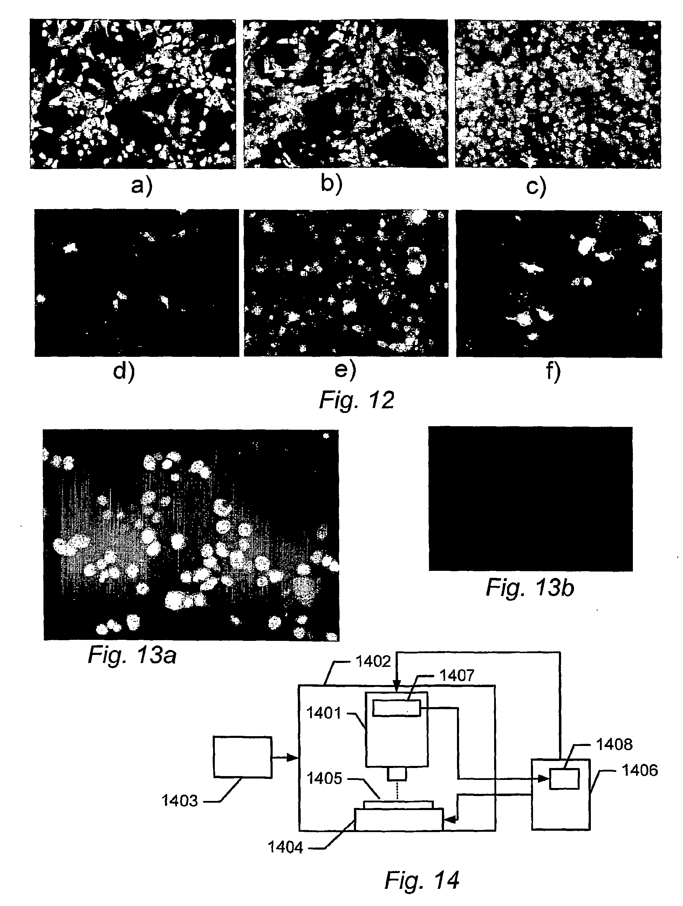

Figs. 12 and 13 illustrate examples of differential cell attachment assays using fluorescent proteins.

As mentioned above, the BSSA described herein can be used in a screening assay for the differential attachment of any two or more different cell types. In one example of such a differential cell attachment (DCA) assay, one cell line is genetically modified to stably express the enhanced yellow fluorescent protein (EYFP) gene whereas the other either expresses (enhanced cyan fluorescent protein (ECFP) or no fluorescent protein at all. The two cell lines are seeded in various proportions on the BSSA wafer. After one to seven days in cell culture the proportion of the two cell types are calculated by fluorescence microscopy. By comparing with the reference tester areas the structures can be examined for advantages in attachment, proliferation etc., as a higher percentage of the relevant cell type is attached to the surface. For example, by use of 4 inch BSSA wafer it is possible to screen approximately 1500 squares with a size of 0.2 mm×0.2 mm, each comprising a different surface structure. Such a square can accommodate several thousand cells, making the quantification of the DCA effect statistically highly relevant.

Fig. 12 shows an example of a differential cell attachment assay where cells are labelled with whole cell labelling. Figs. 12a-c show fibroblasts(NIH) with serum on a tester area with a structure D2/4 as described in connection with fig. 7, 1 day (fig. 12a), 2 days (fig. 12b) and 7 days (fig. 12c) after the cells were seeded on the wafer, respectively. Figs. 12d-f show Osteoblasts (MC3T3) with serum on a tester area with a structure D2/4 as described in connection with fig. 7, 1 day (fig. 12d), 2 days (fig. 12e) and 7 days (fig. 12f) after the cells were seeded on the wafer, respectively.

Fig. 13 shows an example of cells labelled with nuclear localized fluorescent proteins. In particular, fig. 13a shows BOSC 23 cells transiently transfected with nuclear localized ECFP (cyan) seen through a cyan optical filter. In another example, the cells can be transduced with retroviral vectors stably expressing the different fluorescent proteins; or cell clones stably expressing the fluorescent proteins can be generated by isolation of vectors that are stably maintained in the cell e.g. after integration of the vector. Using a yellow filter (fig13 b) no fluorescent background can be detected demonstrating that it is possible to completely discriminate between the enhanced yellow fluorescent protein (EYFP) and ECFP using these filter set. It can furthermore be seen that it is possible to count the number of cells expressing the nuclear localized EYFP and ECFP.

Fig. 14 shows a schematic block diagram of a device for recording results of a screening for a biocompatible surface structure. The device comprises a microscope 1401 placed in an incubator 1402 with an incubator control unit 1403 capable of controlling humidity, CO₂, temperature and/or other parameters inside the incubator. The device further comprises a sample holder 1404 for holding a BSSA wafer 1405 such that the wafer can be inspected by the microscope 1401. The system further comprises a control unit 1406 for controlling the microscope and the position of the wafer relative to the optical axis of the microscope. For example, the sample holder may be movably mounted such that it can be displaced in one or more directions under the control of the control unit 1406, thereby allowing the microscope to acquire images of different individual tester areas in an automatic or semiautomatic fashion. Alternatively or additionally, the microscope 1401 may be mounted movably in one or more directions relative to the sample holder 1404. The microscope 1401 comprises a digital camera 1407 for recording images of the tester areas of the wafer, and the control unit is further adapted to control the camera and the microscope, i.e. functions such as focussing, shutter speed, any optical filtering, etc. To ensure correct focus for each picture in each tester area, the microscope is equipped with a laser focus system ensuring rapid and reliable focusing over the whole wafer independently of the size of the wafer. The control unit 1406 further comprises an image processing unit 1408 adapted to receive the recorded images and to perform an image analysis of the received images. For example, the image analysis may include the detection of cells of different fluorescent colours and the calculation of the ratio of the number of cells of different fluorescent colours. Alternatively or additionally, the image analysis process may include a measurement of the total area of a tester area covered by cells with a given fluorescent colour, or similar image analysis steps. The image analysis program may further perform a sorting of the tester areas, e.g. according to highest percentage of cells with a predetermined property on that area, e.g. cells expressing a yellow fluorescent protein. The general morphology of the cells on the respective tester areas may also be evaluated using differential interference contrast (DIC) imaging.

The control unit may be implemented by hardware comprising several distinct elements, by means of a suitably configured and programmed computer, e.g. a personal computer, or as a combination thereof.

The microscope and the software controlling the microscope and performing the image analysis is adapted to automatically process wafers of one or more different sizes, e.g. wafers up to at least 6000 tester squares of a size of 2x2 mm. For each tester square one to several pictures are obtained. The microscope, the sample holder, and the control unit are further adapted to process wafers of different sizes, e.g. 4 inch wafers, 6 inch wafers, 8 inch wafers, and/or 12 inch wafers, in an automated fashion.

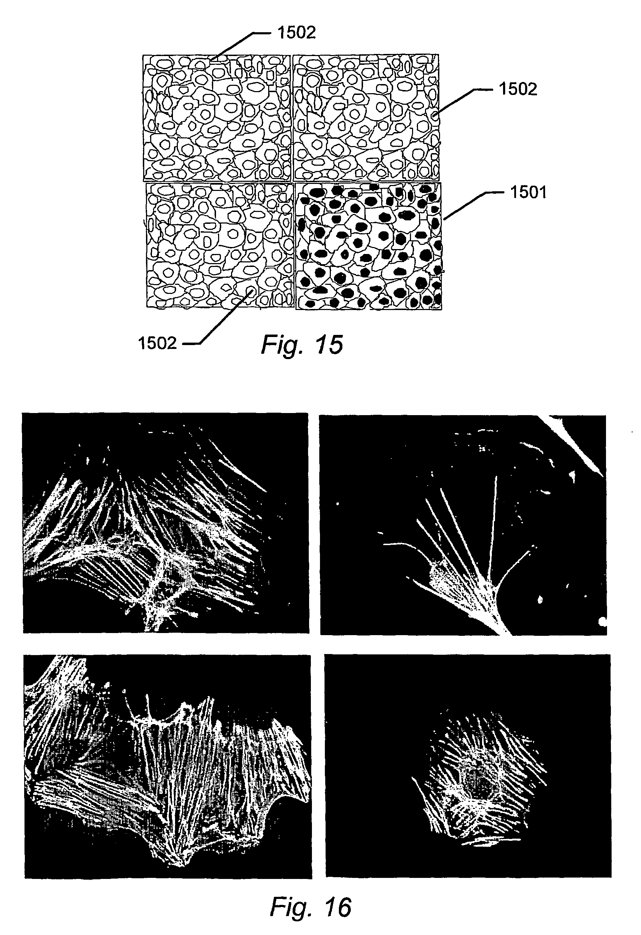

Fig. 15 illustrates an example of a gene induction assay. This example of a gene induction assay uses primary cells from Knock-in mice. For live cells reporter systems using EGFP or other fluorescent protein expressing reporter systems can be used to construct reporter constructs to replace naturally occurring genes. After homologous recombination in ES cells knock in mice are generated, e.g. as described in "Dmd(mdx-beta geo): a new allele for the mouse dystrophin gene" by K. Wertz and EM. Fuchtbauer, Dev Dyn. 1998 Jun;212(2):229-41. From these mice relevant primary cells are isolated. Cells from such mice express the EGFP construct when the targeted gene is induced. Fig 15 shows 4 tester areas of a BSSA wafer as an example of such a screening for an osteoinductive surface. The tester area 1501 an osteoinductive surface while tester areas 1502 comprise non osteoinductive surfaces. It is understood that a variety of other reporter systems can be used in this setup. One example is a Betagal expressing Knock-in mice. A collection of more than 6000 different Knock-in mice exist using this expressing system as described in "A large-scale, gene-driven mutagenesis approach for the functional analysis of the mouse genome" by Hansen J, Floss T, Van Sloun P, Fuchtbauer EM, Vauti F, Arnold HH, Schnutgen F, Wurst W, von Melchner H, Ruiz P, Proc Natl Acad Sci U S A, 2003 Aug 19;100(17):9918-22. Epub 2003 Aug 6.

### Example 2

### Kit comprising a BSSA wafer for cell screening

A kit for screening for insulin producing cells comprises different BSSA wafers, and may further include standard cell culture media and sterile containers as well as various primary cells generated from an insulin Knock-in mouse. By using such cells, surfaces can be assayed for their capability to differentiate e.g. mesenchymal stem cells into beta-cells (insulin producing cells), it can be used to assay for preferential attachment and expansion of beta-cells etc. For human primary cells, cells can e.g. be fixed and stained with antibodies for the relevant genes. The above kit may be used in connection with a microscope described herein. Alternatively or additionally, the kit may comprise such a microscope and/or a software package with control software and/or analysis software for automatic or semiautomatic screening of the wafers.

A BSSA described herein may be used for the screening of biocompatible surfaces in connection with a number of diseases:

For example, any structure that can direct a cell type to the formation of dopaminergenic neurons are of interest as a direct treatment of Parkinson disease as well as they are of potential interest for a variety of other nerve related diseases like Alzheimer. Cells maintained in such a differentiation state further are of great interest, because they can be utilized for screening as well as initial drug testing purposes. The efficiency of cell type attachment to the different surface structures can be measured using immunostaining techniques using commercially available antibodies that recognise cell type specific protein markers, e.g. synapsin I (neurons), GFAP (astrocytes) and PLP (oligodendrocytes), Glia with different coloured fluorescent tags and identify the different cell types by double immunostaining and fluorescence microscopic technologies. The presence of different cell types can be measured at different time intervals which also allows an identification of surface micro- and/or nanometer scale structures that support expansion of neuronal populations.

DAergic neurons can be identified by immunostaining using antibodies specific for tyrosine hydroxylase (TH) and the dopamine transporter (DAT). The ability of the surface structures to support the presence and differentiation of DAergic neuron relative to other cell types, e.g. other neuronal types and astrocytes, can be measured.

Furthermore, any structure that facilitates expansion of Beta-cells or induces differentiation of stem (mesenchymal) cells into insulin producing beta-cells will be of interest in diabetes. Such cells can be used to treat the growing proportion of the population that develops type I diabetes due to lifestyle and/or old age. Insulin producing cells grown on such a surface may be used for drug testing/validation assays.

Structures that enable and/or facilitate the isolation, expansion, priming or differentiation of cells from bone marrow aspirates for bone forming capabilities can be identified by using BSSA. Cells grown on such structures can be used in conjunction with orthopaedic surgery. Primary cells grown on such structures can be used for various drug testing applications.

It is further desirable in a variety of medical applications to be able to transfect primary cells without the use of viral transduction. The BSSA described herein can be applied to screen for nano- and/or micrometer scale structures that either alone or in conjunction with different coatings (e.g. PLA, PLG, PAGA, and poly (D,L-lactide-co-glycolide) (PLGA) polymers) will increase the uptake of DNA in various cell types. For example, the reporter system may consist of a mammalian DNA expression vector encoding enhanced green fluorescent protein (EGFP). This plasmid is applied to the BSSA and structures of interest are identified by fluorescent microscopy.

Such structures will be of major interest in siRNA technologies as well as gene transduction of primary cells for transient expression of an effector gene e.g. BMP-2 in bone forming cells used in orthopaedic research

### Example 3

### Screening of surfaces with embryonic fibroblasts and Osteogenic MC3T3 cells:

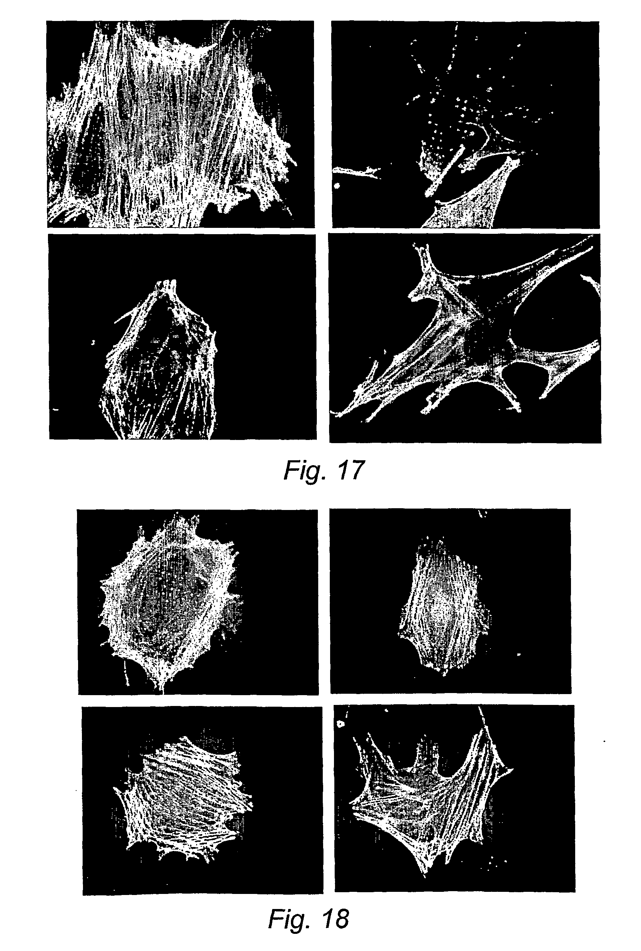

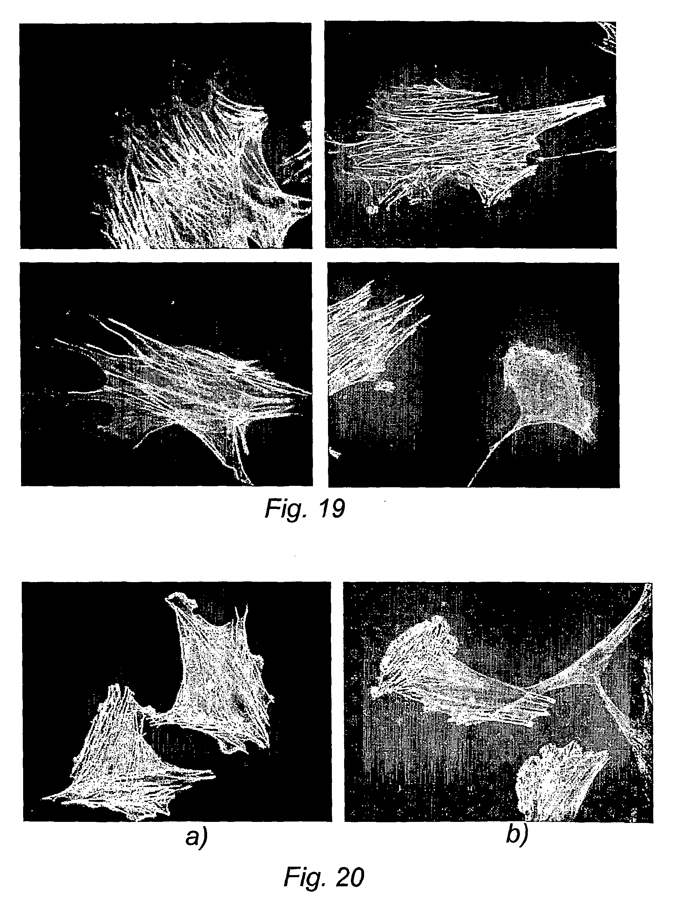

Figs. 16- 19 show screening results of embryonic fibroblasts on tester areas of a BSSA wafer: Fig. 16 shows embryonic fibroblasts on a reference surface of a BSSA wafer. Fig. 17 shows embryonic fibroblasts on a tester area having a "D2/4" surface structure with a feature height of 1600nm. Fig. 18 shows further examples of embryonic fibroblasts on a tester area having a "D2/4" surface structure with a feature height of 1600nm. Fig. 19 shows embryonic fibroblasts on a tester area having a "D2/10" surface structure with a feature height of 1600nm.

Embryonic fibroblasts are large stretched cells with long and thick stress fibres (actin filament bundles). Furthermore, embryonic fibroblasts are motile cells. As can be seen from figs. 16-19, the stress fibres have a tendency to react to the D2/4 but not upon the D2/10 structure by selectively organizing along them. In less motile cells, deposits of F-actin are found around the structures.

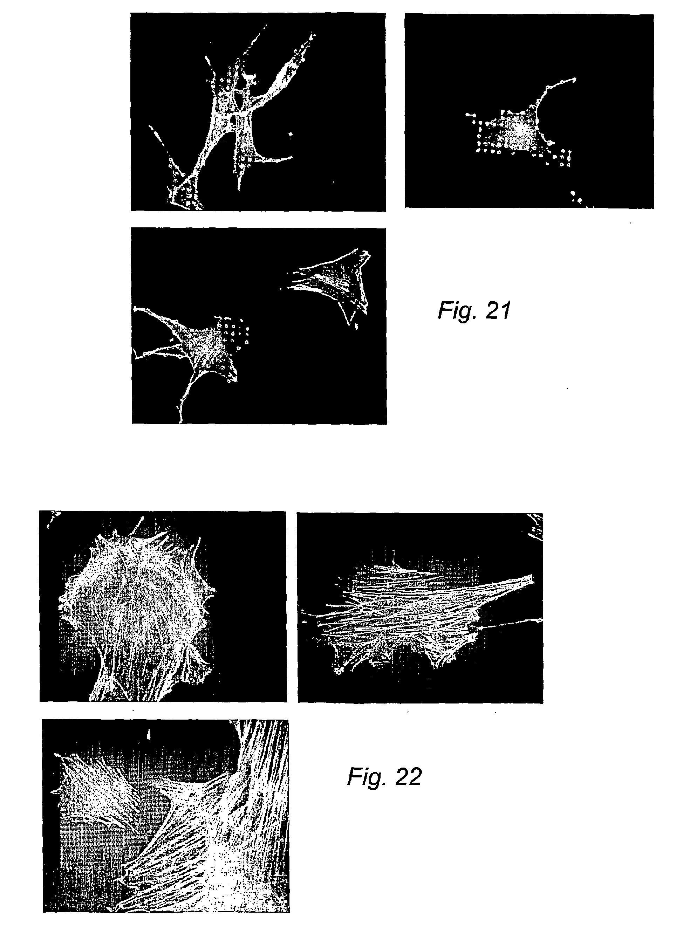

Figs. 20-22 show screening results of Osteogenic MC3T3 cells on tester areas a BSSA wafer: Fig. 20a shows MC3T3 cells on glass, and Fig. 20a shows MC3T3 cells on a reference surface of a BSSA wafer. Fig. 21 shows MC3T3 cells on a tester area having a "D2/4" surface structure (1600nm). Fig. 22 shows MC3T3 cells on a tester area having a "D2/10" surface structure (1600nm).

MC3T3 are smaller cells with thinner stress fibres. Furthermore, MC3T3 are less motile cells. As can be seen from figs. 20-22, there are less stress fibres on D2/4 but not on D2/10 structures. Furthermore, there are many F-actin deposits around the D2/4 structures. Finally, the cells shape according to the structures on which they are placed.

All stainings in figs. 16-22 were done with Rhodamin labeled Phalloidine 48 hrs after seeding the cells.

## Claims

1. A biosurface structure array for testing different microenvironments for cultivation of cells or organisms, the biosurface structure array comprising a substrate layer on which a plurality of tester areas are arranged, wherein each tester area has:
a. a surface area of at least 0.1225 mm², wherein each linear dimension is at least 350 µm, and
b. a surface to be exposed to said cells or organisms, wherein the surface has a respective predetermined regular nano- or micrometer scale topographical structure.

2. A biosurface structure array according to claim 1, wherein the regular structure Is a periodic structure.

3. A biosurface structure array for screening cells, the biosurface structure array comprising a plurality of tester areas wherein each tester area has a surface to be exposed to said cells, wherein the surface has a predetermined surface area and a predetermined periodic topographical structure.

4. A biosurface structure array according to any one of claims 1-3, wherein the surface area is at least 0.09 mm², and wherein each linear dimension is at least 300 µm.

5. A biosurface structure array according to any one of claims 1-3, wherein the surface area is at least 4 mm², and wherein each linear dimension is at least 2 mm.

6. A biosurface structure array according to any one of claims 1-5, wherein said topographical structure comprises a plurality of features whose lateral (pitch) dimensions are between about 1 nm and about 1000,000nm.

7. A biosurface structure array according to claim 6, wherein said dimensions are selected from one of the intervals: between about 1 nm and about 2nm, between about 2nm and about 4nm, between about 4nm and about 10 nm, between about 10nm, and about 100 nm, between about 100nm and about 1000 nm, between about 1 µm and about 2 µm, between about 2 µm and about 4 µm, between about 4 µm and about 10 µm, and between about 10 µm and about 100 µm.

8. A biosurface structure array according to any one of claims 1-7, wherein the substrate layer of said array comprises silicon.

9. A biosurface structure array according to any one of claims 1-7, wherein the substrate layer of said array comprises at least one of a semiconductor, a single metal, an alloy, a ceramic, a polymer, a copolymer, a composite, a drug delivery system, a bioactive compound.

10. A biosurface structure array according to any one of claims 1-9, wherein the surface layer of said array comprises a metal, such as tantalum, titanium, a Ti-Al-V alloy, gold, chromium, a metal oxide, a semiconductor oxide, a metal nitride, a semiconductor nitride, a polymer, a biopolymer, an alloy.

11. A biosurface structure array according to any one of claims 1-10, wherein one or more bioactive compound is deposited on a surface layer of said array.

12. A biosurface structure array according to claim 11, wherein said bioactive compound is a polymer comprising a polypeptide.

13. A biosurface structure array according to claim 92, wherein said polymer includes at least one of an antibody, antigen, glycoprotein, lipoprotein, DNA, RNA, polysaccharide, lipid, organic compound, inorganic compound.

14. A biosurface structure array according to any one of claims 1-13, wherein each tester area has a largest and a smallest linear dimension, and the ratio between the largest and the smallest linear dimension is smaller than 10, preferably, smaller than 5, more preferably smaller than 2.

15. A kit comprising a biosurface structure array according to any one of claim 1-14.

16. A kit according to claim 15 for use in at least one of a cell attachment assay, a cell differentiation assay, a cell motility assay, a cell proliferation assay.

17. A method of screening for a biocompatible surface structure comprising the step of:
a. combining a biosurface structure array according to any one of claims 1-14, with isolated cells or organisms under conditions suitable for the maintenance and/or growth of said cells or organisms.

18. A method according to claim 17, further comprising the step of incubating (a) under conditions suitable for the maintenance and/or growth and/or differentiation /dedifferentiation of said cells or organisms.

19. A method according to claim 17 or 18, wherein each linear dimension of the tester area is large enough to accommodate a row of at least 5 cells, preferably at least 10 cells or organisms, more preferably at least 20 cells or organisms.

20. A method according to any one of claims 17-19, further comprising the step of comparing the frequency of attachment of said cells or organisms to any one tester area of said biosurface structure array.

21. A method according to any one of claims 17-19, further comprising the step of comparing spreading of said cells or organisms on any one tester area of said biosurface structure array.

22. A method according to any one of claims 17-19, further comprising the step of comparing differentiation of cells or organisms attached to any one tester area of said biosurface structure array.

23. A method according to any one of claims 17-22, wherein said cells or organisms include at least one of animal cells, mammalian cells, plant cells, yeast cells, eukaryotic or prokaryotic cells or organisms.

24. A biocompatible material having a surface, wherein at least a part of said surface is **characterized by** a predetermined regular nano- or micrometer scale topographical structure, the surface being obtained by the method according to any one of claims 17-23.

25. A wafer comprising a biosurface structure array according to any one of claims 1-14.

26. Use of a wafer according to claim 25, in an *in vitro* cell bioassay.

27. A kit comprising the wafer according to claim 25 for performing an *in vitro* cell bioassay.

## Patentansprüche

1. Oberflächenstruktur-Array zur Prüfung verschiedener Mikroumgebungen zur Kultivierung von Zellen oder Organismen, wobei das Oberflächenstruktur-Array eine Substratschicht umfasst, auf welcher eine Mehrheit von Prüfbereichen angeordnet ist, wobei jeder Prüfbereich aufweist:
a. einen Oberflächenbereich von mindestens 0,1125 mm², wobei jede lineare Dimension mindestens 350 µm beträgt, und
b. eine den Zellen oder Organismen auszusetzende Oberfläche, wobei die Oberfläche eine jeweilige vorgegebene, reguläre topographische Struktur auf Nano- oder Mikrometerskala aufweist.

2. Oberflächenstruktur-Array nach Anspruch 1, wobei die reguläre Struktur eine periodische Struktur ist.

3. Oberflächenstruktur-Array zum Screening von Zellen, welches eine Mehrheit von Prüfbereichen umfasst, wobei jeder Prüfbereich eine den Zellen auszusetzende Oberfläche aufweist, wobei die Oberflache einen vorgegebenen Oberflächenbereich und eine vorgegebene, periodische topographische Struktur aufweist.

4. Oberflächenstruktur-Array nach einem der Ansprüche 1 bis 3, wobei der Oberflächenbereich mindestens 0,09 mm² beträgt, und wobei jede lineare Dimension mindestens 300 µm beträgt.

5. Oberflächenstruktur-Array nach einem der Ansprüche 1 bis 3, wobei der Oberflächenbereich mindestens 4 mm² beträgt, und wobei jede lineare Dimension mindestens 2 mm beträgt.

6. Oberflächenstruktur-Array nach einem der Ansprüche 1 bis 5, wobei die topographische Strukur eine Mehrheit von Merkmalen umfasst, deren seitliche (Pitch-) Dimensionen zwischen etwa 1 nm und etwa 1000.000 nm betragen.

7. Oberflächenstruktur-Array nach Anspruch 6, wobei die Dimensionen aus einem der folgenden Intervallen ausgewählt sind: zwischen etwa 1 nm und etwa 2 nm, zwischen etwa 2 nm und etwa 4 nm, zwischen etwa 4 nm und etwa 10 nm, zwischen etwa 10 nm und etwa 100 nm, zwischen etwa 100 nm und etwa 1000 nm, zwischen etwa 1 µm und etwa 2 µm, zwischen etwa 2 µm und etwa 4 µm, zwischen etwa 4 µm und etwa 10 µm, und zwischen etwa 10 µm und etwa 100 µm.

8. Oberflächenstruktur-Array nach einem der Ansprüche 1 bis 7, wobei die Substratschicht des Arrays Silizium umfasst.

9. Oberflächenstruktur-Array nach einem der Ansprüche 1 bis 7, wobei die Substratschicht des Arrays mindestens eines aus einem Halbleiter, einem einzigen Metall, einer Legierung, einer Keramik, einem Polymer, einem Copolymer, einem Verbundwerkstoff, einem System zur Abgabe von Arzneimitteln, einer bioaktiven Verbindung umfasst.

10. Oberflächenstruktur-Array nach einem der Ansprüche 1 bis 9, wobei die Oberflächenschicht des Arrays ein Metall, wie beispielsweise Tantal, Titan, eine Ti-Al-V-Legierung, Gold, Chrom, ein Metalloxid, ein Halbleiteroxid, ein Metallnitrid, ein Halbleiternitrid, ein Polymer, ein Biopolymer, eine Legierung, umfasst.

11. Oberflächenstruktur-Array nach einem der Ansprüche 1 bis 10, wobei eine oder mehrere bioaktive Verbindung(en) auf einer Oberflächenschicht des Arrays abgelagert sind.

12. Oberflächenstruktur-Array nach Anspruch 11, wobei die bioaktive Verbindung ein Polymer umfassend ein Polypeptid ist.

13. Oberflächenstruktur-Array nach Anspruch 12, wobei das Polymer mindestens einen/eine/eines von einem Antikörper, Antigen, Glycoprotein, Lipoprotein, einer DNA, RNA, einem Polysaccharid, Lipid, einer organischen Verbindung, einer anorganischen Verbindung umfasst.

14. Oberflächenstruktur-Array nach einem der Ansprüche 1 bis 13, wobei jeder Prüfbereich eine größte und eine kleinste lineare Dimension aufweist, und das Verhältnis der größten zur kleinsten linearen Dimension kleiner als 10, bevorzugt kleiner als 5, mehr bevorzugt kleiner als 2 ist.

15. Kit umfassend ein Oberflächenstruktur-Array nach einem der Ansprüche 1 bis 14.

16. Kit nach Anspruch 15 zur Verwendung in mindestens einem von einem Zellenanhaftungsassay, einem Zellendifferenzierungsassay, einem Zellenmotilitätsassay, einem Zellenproliferationsassay.

17. Verfahren zum Screening einer biokompatiblen Oberflächenstruktur umfassend den folgenden Schritt:
a. Kombinieren eines Oberflächenstruktur-Arrays nach einem der Ansprüche 1 bis 14 mit isolierten Zellen oder Organismen unter zur Aufrechterhaltung und/oder zum Wachstum der Zellen oder Organismen geeigneten Bedingungen.

18. Verfahren nach Anspruch 17, ferner umfassend den Schritt des Inkubierens (a) unter zur Aufrechterhaltung und/oder zum Wachstum und/oder zur Differenzierung/Dedifferenzierung der Zellen oder Organismen geeigneten Bedingungen.

19. Verfahren nach Anspruch 17 oder 18, wobei jede lineare dimension des Prüfbereiches zur Aufnahme einer Reihe von mindestens 5 Zellen, bevorzugt mindestens 10 Zellen oder Organismen, mehr bevorzugt mindestens 20 Zellen oder Organismen, ausreichend groß ist.

20. Verfahren nach einem der Ansprüche 17 bis 19, ferner umfassend den Schritt des Vergleichens der Anhaftungsfrequenz der Zellen oder Organismen an jeglichem Prüfbereich des Oberflächenstruktur-Arrays.

21. Verfahren nach einem der Ansprüche 17 bis 19, ferner umfassend den Schritt des Vergleichens der Ausbreitung der Zellen oder Organismen auf jeglichem Prüfbereich des Oberflächenstruktur-Arrays.

22. Verfahren nach einem der Ansprüche 17 bis 19, ferner umfassend den Schritt des Vergleichens der Differenzierung von an jeglichem Prüfbereich des Oberflächenstruktur-Arrays angehafteten Zellen oder Organismen.

23. Verfahren nach einem der Ansprüche 17 bis 22, wobei die Zellen oder Organismen mindestens eines von tierischen Zellen, Säugerzellen, pflanzlichen zellen, Hefezellen, eukaryotischen oder prokaryotischen Zellen oder Organismen umfassen.

24. Biokompatibles Material, welches eine Oberfläche aufweist, wobei mindestens ein Teil der Oberfläche durch eine vorgegebene, reguläre topographische Struktur auf Nano- oder Mikrometerskala **gekennzeichnet** ist, wobei die Oberfläche durch das Verfahren nach einem der Ansprüche 17 bis 23 bereitgestellt ist.

25. Wafer umfassend ein Oberflächenstruktur-Array nach einem der Ansprüche 1 bis 14.

26. Verwendung eines Wafers nach Anspruch 25 in einem *in-vitro* Zellen-Bioassay.

27. Kit umfassend den Wafer nach Anspruch 25 zur Durchführung eines *in-vitro* Zellen-Bioassays.

## Revendications

1. Réseau de structure de biosurface destiné à tester différents micro-environnements pour la culture des cellules ou des organismes, le réseau de structure de biosurface comprenant une couche de substrat, sur laquelle une pluralité de zones de test est disposée, dans lequel chaque zone de test présente:
a. une aire de surface d'au moins 0,1225 mm², dans laquelle chaque dimension linéaire est d'au moins 350 µm, et
b. une surface destinée à être exposée auxdites cellules ou auxdits organismes, où la surface présente une structure respective, régulière et prédéterminée de topographie à l'échelle nanométrique ou micrométrique.

2. Réseau de structure de biosurface selon la revendication 1, dans lequel la structure régulière est une structure périodique.

3. Réseau de structure de biosurface pour le criblage des cellules, le réseau de structure de biosurface comprenant une pluralité de zones de test, où chaque zone de test présente une surface destinée à être exposée auxdites cellules, où la surface présente une aire de surface prédéterminée et une structure topographique périodique et prédéterminée.

4. Réseau de structure de biosurface selon l'une quelconque des revendications 1 à 3, dans lequel l'aire de surface est au moins 0,09 mm², et dans lequel chaque dimension linéaire est au moins 300 µm.

5. Réseau de structure de biosurface selon l'une quelconque des revendications 1 à 3, dans lequel l'aire de surface est au moins 4 mm², et dans lequel chaque dimension linéaire est au moins 2 mm.

6. Réseau de structure de biosurface selon l'une quelconque des revendications 1 à 5, dans lequel ladite structure de topographie comprend une pluralité de caractéristiques dont les dimensions latérales (de pas) sont comprises entre environ 1 nm et environ 1000.000nm.

7. Réseau de structure de biosurface selon la revendication 6, dans lequel lesdites dimensions sont sélectionnées de l'un des intervalles: compris entre environ 1 nm et environ 2nm, entre environ 2nm et environ 4nm, entre environ 4nm et environ 10nm, entre environ 10nm et environ 100nm, entre environ 100nm et environ 1000nm, entre environ 1 µm et environ 2 µm, entre environ 2 µm et environ 4 µm, entre environ 4 µm et environ 10 µm, et entre environ 10 µm et environ 100 µm.

8. Réseau de structure de biosurface selon l'une quelconque des revendications 1 à 7, dans lequel la couche de substrat dudit réseau comprend le silicium.

9. Réseau de structure de biosurface selon l'une quelconque des revendications 1 à 7, dans lequel la couche de substrat dudit réseau comprend au moins un semi-conducteur, un seul métal, un alliage, un céramique, un polymère, un copolymère, un composite, un système de délivrance de médicament ou un composé bioactif.

10. Réseau de structure du biosurface selon l'une quelconque des revendications 1 à 9, dans lequel la couche de surface dudit réseau comprend un métal, tel que tantale, titane, un alliage de Ti-Al-V, or, achrome, un oxyde métallique, un oxyde de semi-conducteur, un nitrure métallique, un nitrure de semi-conducteur, un polymère, un biopolymère, un alliage.

11. Réseau de structure de biosurface selon l'une quelconque des revendications 1 à 10, dans lequel un ou plusieurs composés bioactifs sont déposés sur une couche de surface dudit réseau.

12. Réseau de structure de biosurface selon la revendication 11, dans lequel ledit composé bioactif est un polymère comprenant un polypeptide.

13. Réseau de structure de biosurface selon la revendication 12, dans lequel ledit polymère comprend au moins un anticorps, un antigène, une glycoprotéine, une lipoprotéine, un ADN, un ARN, un polysaccharide, un lipide, un composé organique ou un composé inorganique.

14. Réseau de structure de biosurface selon l'une quelconque des revendications 1 à 13, dans lequel chaque zone de test présente une dimension linéaire la plus grande et la plus petite, et le rapport entre la dimension linéaire la plus grande et la plus petite est inférieur à 10, préférablement inférieur à 5, plus préférablement inférieur à 2.

15. Trousse comprenant un réseau de structure de biosurface selon l'une quelconque des revendications 1 à 14.

16. Trousse selon la revendication 15 pour l'usage dans au moins une analyse d'attachement de cellule, une analyse de différentiation de cellule, une analyse de motilité de cellule ou une analyse de prolifération cellulaire.

17. Procédé pour le criblage d'une structure de surface biocompatible comprenant l'étape de:
a. combinant un réseau de structure de biosurface selon l'une quelconque des revendications 1 à 14, avec les cellules ou les organismes isolés dans des conditions appropriées pour la maintenance et/ou la croissance desdites cellules ou desdits organismes.

18. Procédé selon la revendication 17, comportant en outre l'étape d'incuber (a) dans des conditions appropriées pour la maintenance et/ou la croissance et/ou la différenciation/dédifférenciation desdites cellules ou desdits organismes.

19. Procédé selon la revendication 17 ou 18, dans lequel chaque dimension linéaire de la zone de test est suffisamment grande pour loger une rangée d'au moins 5 cellules, préférablement d'au moins 10 cellules ou organismes, plus préférablement d'au moins 20 cellules ou organismes.

20. Procédé selon l'une quelconque des revendications 17 à 19, comportant en outre l'étape de comparer la fréquence de l'attachement desdites cellules ou desdits organismes à toute zone de test dudit réseau de structure de biosurface.

21. Procédé selon l'une quelconque des revendications 17 à 19, comportant en outre l'étape de comparer l'étalement desdites cellules ou desdits organismes sur toute zone de test dudit réseau de structure de biosurface.

22. Procédé selon l'une quelconque des revendications 17 à 19, comportant en outre l'étape de comparer la différenciation des cellules ou des organismes fixés à toute zone de test dudit réseau de structure de biosurface.

23. Procédé selon l'une quelconque des revendications 17 à 22, dans lequel lesdites cellules ou lesdits organismes comprennent au moins une cellule animale, une cellule de mammifère, une cellule de plante, une cellule de levure ou une cellule ou un organisme eucaryote ou procaryote.

24. Matériau biocompatible ayant une surface, dans lequel au moins une partie de ladite surface est **caractérisée par** une structure régulière et prédéterminée de topographie à l'échelle nanométrique ou micrométrique, la surface étant obtenue par le procédé selon l'une quelconque des revendications 17 à 23.

25. Wafer comprenant un réseau de structure de biosurface selon l'une quelconque des revendications 1 à 14.

26. Utilisation d'un wafer selon la revendication 25 dans un essai biologique in vitro de cellule.

27. Trousse comprenant le wafer selon la revendication 25 pour effectuer un essai biologique in vitro de cellule.
